Europäisches Patentamt

European Patent Office

Office européen des brevets

(11) Veröffentlichungsnummer : **0 126 341**
**B1**

(12) **EUROPÄISCHE PATENTSCHRIFT**

(45) Veröffentlichungstag der Patentschrift :
01.02.89

(51) Int. Cl.⁴ : **C 07 C 69/54**, C 07 C 67/08,
C 07 C 67/26, C 09 D 3/80

(21) Anmeldenummer : 84104853.1

(22) Anmeldetag : 30.04.84

(54) Verfahren zur Herstellung von (Meth)-acrylsäureestern und deren Verwendung.

(30) Priorität : 06.05.83 DE 3316593

(43) Veröffentlichungstag der Anmeldung :
28.11.84 Patentblatt 84/48

(45) Bekanntmachung des Hinweises auf die Patenterteilung : 01.02.89 Patentblatt 89/05

(84) Benannte Vertragsstaaten :
BE CH DE FR GB IT LI NL SE

(56) Entgegenhaltungen :
EP--A-- 0 054 105
EP--A-- 0 127 766
BE--A-- 681 520
DE--A-- 1 248 660
DE--A-- 2 003 579
DE--A-- 2 758 019
FR--A-- 2 029 567
FR--A-- 2 316 253
GB--A-- 1 400 978
US--A-- 4 330 643
Die Akte enthält technische Angaben, die nach dem
Eingang der Anmeldung eingereicht wurden und die
nicht in dieser Patentschrift enthalten sind.

(73) Patentinhaber : BASF Aktiengesellschaft
Carl-Bosch-Strasse 38
D-6700 Ludwigshafen (DE)

(72) Erfinder : Schornick, Gunnar, Dr.
Konrad-Adenauer-Strasse 8
D-6719 Neuleiningen (DE)
Erfinder : Buethe, Ingolf, Dr.
Am Wasserturm 1
D-6737 Boehl-Iggelheim (DE)
Erfinder : Jacobi, Manfred, Dr.
Heidelberger Ring 32 b
D-6710 Frankenthal (DE)
Erfinder : Lenz, Werner, Dr.
Heinrich-Baermann-Strasse 14
D-6702 Bad Duerkheim (DE)
Erfinder : Lehnerer, Wolfgang, Dr.
Brunckstrasse 23
D-6710 Frankenthal (DE)

Anmerkung : Innerhalb von neun Monaten nach der Bekanntmachung des Hinweises auf die Erteilung des
europäischen Patents im Europäischen Patentblatt kann jedermann beim Europäischen Patentamt gegen das erteilte
europäische Patent Einspruch einlegen. Der Einspruch ist schriftlich einzureichen und zu begründen. Er gilt erst als
eingelegt, wenn die Einspruchsgebühr entrichtet worden ist (Art. 99(1) Europäisches Patentübereinkommen).

**Beschreibung**

Die vorliegende Erfindung betrifft ein Verfahren zur Herstellung von Estern der Acryl- oder Methacrylsäure mit hydroxylgruppenhaltigen Polyestern oder Polyethern und deren Verwendung zur Herstellung strahlungshärtbarer Überzugsmassen, z. B. in Lack-Formulierungen, welche durch UV- bzw. Elektronenstrahlen schnell gehärtet werden können.

Mit aktinischer Strahlung härtbare Bindemittel auf Basis von linearen oder verzweigten Polyestern sind bekannt, z. B. aus FR-A 2 029 567, DE-OS 28 38 691, DE-OS 31 06 570, EP-A 2866 und EP-A 54105. In allen diesen Fällen werden die für die Polymerisation erforderlichen Doppelbindungen durch Umsetzung der Hydroxylgruppen von linearen oder verzweigten Polyestern mit (Meth-)Acrylsäure eingeführt.

Die Schwierigkeit besteht in der Entfernung der nicht umgesetzten Acrylsäure aus dem Reaktionsgemisch. Hierzu sind beispielsweise komplizierte Waschvorgänge notwendig, wie sie in der FR-A 2 029 567, der DE-OS 28 38 691 und der DE-OS 31 06 570 vorgeschlagen werden. Einen anderen Weg beschreibt die EP-A 2866, wonach zur Aufarbeitung des Reaktionsgemisches eine Vakuumdestillation durchgeführt wird. Eine weitere Möglichkeit wird in der EP-A 54105 aufgezeigt. Nach dem Veresterungsschritt wird die restliche Acrylsäure mit einer der Säuremenge äquivalenten Menge einer Mono- oder Diepoxidverbindung unter Verwendung von Triphenylphosphin als Katalysator umgesetzt. Die Tatsache, daß die zur Veresterung eingesetzte Acrylsäure im Unterschuß vorliegt (max. 90 Mol.%, bezogen auf die Hydroxylgruppen des Polyesters) und zusätzlich nur teilweise in dieser ersten Stufe umgesetzt wird, setzt die Verwendung von Polyesterolen mit einer höheren Anzahl an Hydroxylgruppen voraus. Diese Maßnahmen führen sowohl beim Ausgangspolyester als auch bei dem daraus hergestellten Polyesteracrylat zu höheren Produktviskositäten.

Aufgabe der vorliegenden Erfindung ist es, ein Verfahren zur Herstellung von Estern der (Meth)-acrylsäure mit hydroxylgruppenhaltigen Polyestern oder Polyethern aufzuzeigen, das die oben geschilderten Nachteile der Verfahren des bekannten Standes der Technik nicht aufweist und insbesondere hinsichtlich benötigter Cycluszeit und Ausnutzung der Einsatzstoffe deutliche Vorteile zeigt und möglichst universell verwendbar ist.

Gegenstand der vorliegenden Erfindung ist ein Verfahren zur Herstellung von Estern der Acryl- oder Methacrylsäure mit hydroxylgruppenhaltigen organischen Verbindungen, das dadurch gekennzeichnet ist, daß man gesättigte, gegebenenfalls Ethergruppen enthaltende Polyester, die mindestens 2 freie Hydroxylgruppen pro Molekül enthalten, oder Polyether, die mindestens 2 freie Hydroxylgruppen pro Molekül enthalten, wobei diese Polyester oder Polyether durchschnittliche Molekulargewichte $\overline{M_n}$ zwischen 400 und 4 000 aufweisen, mit 100 bis 150 Mol.%, bezogen auf die OH-Gruppen des Polyesters bzw. Polyethers, Acrylsäure oder Methacrylsäure in Gegenwart eines sauren Veresterungskatalysators und mindestens eines Kohlenwasserstoffs, der mit Wasser ein azeotropes Gemisch bildet sowie geringer Mengen eines Polymerisationsinhibitors unter azeotroper Entfernung des entstehenden Wassers bei erhöhter Temperatur verestert, nach der Veresterung den Kohlenwasserstoff destillativ entfernt und nach Neutralisation des Veresterungskatalysators die restliche Acryl- oder Methacrylsäure mit einer der Säurezahl äquivalenten Menge einer mindestens zwei Epoxidgruppen pro Molekül aufweisenden Epoxidverbindung bis zu einer Säurezahl $\leqslant 5$ mg KOH/g umsetzt.

Durch das erfindungsgemäße Verfahren werden die Nachteile der zuvor genannten Verfahren des bekannten Standes der Technik vermieden. So lassen sich nach dem erfindungsgemäßen Verfahren beispielsweise auch solche Polyester- und Polyetheracrylate herstellen, bei denen aufgrund ihrer Polarität ein Auswaschen der überschüssigen Acrylsäure mit Wasser oder wäßrigen Lösungen wegen irreversibler Emulsionsbildung nicht möglich ist. Da der Umsatz der Hydroxylgruppen der Polyester bzw. Polyether mit (Meth)-acrylsäure im allgemeinen größer als 85 %, vorzugsweise größer als 90 % ist, sind Funktionalitäten $\leqslant 3$, d. h. maximal 3 OH-Gruppen pro Polyolmolekül im allgemeinen ausreichend, um mit den erfindungsgemäßen Produkten hohe Härtungsgeschwindigkeiten bei gleichzeitig guter Kratzfestigkeit der Überzüge zu erzielen. Der geringe Anteil an nichtumgesetzten Hydroxylgruppen führt außerdem zu niedrigviskosen Bindemitteln und verringert die Wasserempfindlichkeit der daraus hergestellten Überzüge. Damit verbunden ist auch ein geringer Monomerenbedarf, um die gewünschten Verarbeitungsviskositäten einzustellen.

Zu den für das erfindungsgemäße Verfahren zu verwendenden einzelnen Komponenten ist folgendes auszuführen :

Als gesättigte, gegebenenfalls Ethergruppen enthaltende Polyester bzw. Polyether, die mindestens zwei freie Hydroxylgruppen pro Molekül enthalten und die durchschnittliche Molekulargewichte $\overline{M_r}$ zwischen 250 und 4 000, vorzugsweise zwischen 450 und 2 000 aufweisen, eignen sich die üblichen.

Derartige hydroxylgruppenhaltige Polyester können z. B. in üblicher Weise durch Veresterung von Dicarbonsäuren mit Diolen und Triolen hergestellt werden. Die Umsetzung kann dabei in Substanz oder in Gegenwart eines Schleppmittels erfolgen. Die Ausgangsstoffe für solche hydroxylgruppenhaltige Polyester (= Polyesterole) sind dem Fachmann bekannt. Bevorzugt können als Dicarbonsäuren Bernsteinsäure, Glutarsäure, Adipinsäure, Sebacinsäure, o-Phthalsäure, deren Isomere und Hydrierungsprodukte sowie veresterbare Derivate, wie Anhydride oder Dialkylester der genannten Säuren eingesetzt werden. Als Diole kommen vorzugsweise in Betracht Ethylenglykol, Propylenglykol-1,2 und -1,3, Butandiol-1,4, Hexandiol-1,6, Neopentylglykol, Cyclohexandimethanol sowie Polyglykole vom Typ des Ethylenglykols und Propylenglykols.

Als Triole sind in erster Linie Trimethylolpropan und Glycerin zu nennen.

Zu den erfindungsgemäß einzusetzenden Polyesterolen zählen auch Polycaprolactondiole und -triole, deren Herstellung dem Fachmann ebenfalls bekannt ist.

Als hydroxylgruppenhaltige Polyether (= Polyetherole) kommen z. B. solche in Frage, welche nach bekannten Verfahren durch Umsetzung von zwei- und/oder mehrwertigen Alkoholen mit verschiedenen Mengen an Ethylenoxid und/oder Propylenoxid erhalten werden können. Bei den Ethylenglykol/Propylenglykol-Mischkondensationsprodukten kann die Umsetzung zweckmäßigerweise so gesteuert werden, daß endständig überwiegend primäre Hydroxylgruppen entstehen. Desgleichen sind auch Polymerisationsprodukte des Tetrahydrofurans oder Butylenoxyds verwendbar. Das durchschnittliche Molekulargewicht $\bar{M}_n$ der Polyetherole soll wie bei den Polyesterolen zwischen 400 und 4 000, vorzugsweise zwischen 450 und 2 000 liegen.

Die hydroxylgruppenhaltigen Polyester bzw. Polyether werden mit 100 bis 150 Mol.%, bezogen auf die Hydroxylgruppen des Polyesters bzw. Polyethers, Acrylsäure und/oder Methacrylsäure versetzt und in Gegenwart eines sauren Veresterungskatalysators, wie z. B. Schwefelsäure oder p-Toluolsulfonsäure, sowie in Gegenwart eines Kohlenwasserstoffs, der mit Wasser ein azeotropes Gemisch bildet, bis zu einem Umsatz von mindestens 85 %, vorzugsweise 90 bis 95 %, der Hydroxylgruppen des Polyesters bzw. Polyethers wie üblich, beispielsweise bei 60 bis 140 °C verestert. Das gebildete Reaktionswasser wird azeotrop entfernt. Als Schleppmittel geeignete Kohlenwasserstoffe sind aliphatische und aromatische, z. B. Alkane und Cycloalkane, wie n-Hexan, n-Heptan und Cyclohexan, Aromaten wie Benzol, Toluol und die Xylol-Isomeren, und sog. Spezialbenzine, welche Siedegrenzen zwischen 70 und 140 °C aufweisen. Das eingesetzte Lösungsmittel wird nach der Veresterung, gegebenenfalls unter vermindertem Druck, aus dem Reaktionsgemisch entfernt.

Zur Vermeidung einer vorzeitigen Polymerisation wird die Veresterung der Hydroxylgruppen des Polyols mit der ungesättigten Säure zweckmäßigerweise in Gegenwart geringer Mengen von Inhibitoren durchgeführt. Dabei handelt es sich um die üblichen, zur Verhinderung einer thermischen Polymerisation verwendeten Verbindungen, z. B. vom Typ des Hydrochinons, der Hydrochinonmonoalkylether, des 2,6-Di-t-butylphenols, der N-Nitrosoamine der Phenothiazine oder der Phosphorigsäureester. Sie werden im allgemeinen in Mengen von 0,001 bis 2,0 %, vorzugsweise in Mengen von 0,005 bis 0,5 %, bezogen auf die Summe von Polyol und (Meth) acrylsäure, eingesetzt.

Nach der Veresterung wird das Lösungsmittel, d. h. der Kohlenwasserstoff, aus dem Reaktionsgemisch destillativ, gegebenenfalls unter vermindertem Druck, entfernt. Der Veresterungskatalysator wird in geeigneter Weise neutralisiert, z. B. durch Zusatz von tertiären Aminen oder Alkalihydroxyden, bevor die Reaktionsmischung mit einer der Säurezahl äquivalenten Menge einer Polyepoxidverbindung versetzt wird. Die Umsetzung der restlichen (Meth) acrylsäure mit der Polyepoxidverbindung erfolgt im allgemeinen bei 90 bis 130, vorzugsweise bei 100 bis 110 °C bis zu einer Säurezahl von ≤ 5 mg KOH/g.

Im Hinblick auf eine gute Lagerstabilität der auf diese Weise erhaltenen Reaktionsprodukte hat sich eine Katalysierung mit stickstoffhaltigen Verbindungen wie z. B. tert. Aminen oder mit Lewis-Basen vom Typ des Thiodiglykols als besonders vorteilhaft erwiesen.

Als Epoxidverbindungen mit mindestens zwei, vorzugsweise zwei und drei, Epoxidgruppen pro Molekül kommen z. B. in Betracht epoxidierte Olefine, Glycidylester von gesättigten oder ungesättigten Carbonsäuren oder Glycidylether aliphatischer oder aromatischer Polyole. Bevorzugt sind Epoxidverbindungen mit durchschnittlich drei Epoxidgruppen/Molekül. Derartige Produkte werden im Handel in großer Zahl angeboten. Besonders bevorzugt sind Polyglycidylverbindungen von Bisphenol A-Typ und Glycidylether mehrfunktioneller Alkohole, z. B. des Butandiols, des Glycerins und des Pentaerythrits. Beispiele für derartige Polyepoxidverbindungen sind ®Epikote 812 (Epoxidwert : ca. 0,67) und Epikote 828 (Epoxidwert : ca. 0,53) und Epikote 162 (Epoxidwert : ca. 0,61) der Firma Shell.

Durch den sehr hohen Veresterungsgrad von vorzugsweise > 95 %, bezogen auf die Hydroxylgruppen des Polyesterols bzw. Polyetherols, weisen die erfindungsgemäß hergestellten Produkte eine geringe Viskosität auf. Über die nach der Entfernung des Lösungsmittels im Veresterungsgemisch verbleibende Acrylsäuremenge läßt sich aufgrund der stöchiometrischen Umsetzung mit den Polyglycidylverbindungen der Epoxidacrylatanteil variieren. Auf diese Weise und zusätzlich durch Auswahl entsprechender Polyepoxidverbindungen kann man die Eigenschaften des entstehenden Bindemittels gezielt variieren.

Die erfindungsgemäß hergestellten Polyolacrylate werden zur Verarbeitung im allgemeinen mit weiteren, aus der Strahlungshärtung bekannten, monomeren Acrylesterverbindungen versetzt. Beispielhaft seien lediglich genannt 4-t-Butylcyclohexylacrylat, Phenoxyethylacrylat, Hexandioldiacrylat, Tripropylenglykoldiacrylat und Trimethylolpropantriacrylat. Die mittels des erfindungsgemäßen Verfahrens hergestellten Überzugsmittel werden zweckmäßigerweise entweder durch Elektronenstrahlen oder nach Zusatz von Photoinitiatoren durch UV-Strahlen vernetzt und ergeben Filme mit Eigenschaften, die den Anforderungen der Praxis voll gerecht werden.

Die folgenden Beispiele dienen der weiteren Erläuterung der Erfindung. Bei den in den Beispielen genannten Teilen und Prozenten handelt es sich, soweit nicht ausdrücklich anders angegeben, um Gewichtsteile bzw. Gewichtsprozente.

Herstellung der Polyesterole

Gemäß der in Tabelle 1 angegebenen Zusammensetzungen werden die Komponenten auf 160 °C aufgeheizt. Anschließend wird die Temperatur stufenweise auf 210 °C gesteigert und die Veresterung unter Anlegen von Vakuum solange fortgeführt, bis eine Säurezahl < 1,5 mg KOH/g erreicht ist.

Tabelle 1

| | | Polyesterole | | | |
|---|---|---|---|---|---|
| | | PES I | PES II | PES III | PES IV |
| Adipinsäure | (Teile) | 780 | 1080 | 876 | 745 |
| Phthalsäureanhydrid | " | 420 | 548 | 444 | 503 |
| Ethylenglykol | " | 600 | 803 | | |
| Neopentylglykol | " | | | 936 | |
| Hexandiol-1,6 | " | | | | 1003 |
| Trimethylolpropan | " | 560 | 496 | 603 | 570 |
| SZ [mg KOH/g] | | 0,6 | 0,7 | 0,9 | 1,3 |
| OHZ [mg KOH/g] | | 320 | 270 | 270 | 246 |

Herstellung der Polyesteracrylate

Die Komponenten werden in den in Tabelle 2 angegebenen Anteilen zusammengegeben und auf 100 bis 110 °C aufgeheizt. Es wird solange das Reaktionswasser ausgekreist, bis die angegebene Menge erreicht ist. Anschließend wird das Schleppmittel bei vermindertem Druck (etwa 50 mbar) abgezogen. Dann neutralisiert man den Veresterungskatalysator mit einem tertiären Amin, setzt die der bestimmten Säurezahl äquivalente Epoxidmenge und den Katalysator zu. Wird ein Alkalihydroxid zur Neutralisation verwendet, so wird die entsprechende wäßrige oder alkoholische Lösung vor dem Abzug des Lösungsmittels zugesetzt. Die Umsetzung des Epoxids mit der überschüssigen Acrylsäure wird bei ca. 110 °C bis zum Erreichen einer Säurezahl < 5 mg KOH/g durchgeführt.

Tabelle 2

| Beispiel Nr. | | 1 | 2 | 3 | 4 | 5 | 6 | 7 |
|---|---|---|---|---|---|---|---|---|
| verwendetes Polyesterol | | PES I | PES II | PES I | PES II | PES II | PES III | PES IV |
| Polyesterol | (Teile) | 1250 | 1250 | 1380 | 1200 | 1360 | 1400 | 1400 |
| Acrylsäure | " | 582 | 582 | 623 | 490 | 518 | 534 | 495 |
| Cyclohexan | " | 916 | 916 | 1002 | 850 | 939 | 967 | 945 |
| H₂SO₄ | " | 5,5 | 5,5 | 6 | 5,1 | 5,6 | 5,8 | 5,7 |
| MEHQ | " | 1,8 | 1,8 | 2,0 | 1,7 | 1,9 | 1,9 | 1,9 |
| Kerobit TBK | " | 0,9 | 0,9 | 1,0 | 0,8 | 0,95 | 0,95 | 0,95 |
| Phenothiazin | " | 0,04 | 0,04 | 0,05 | 0,04 | 0,05 | 0,05 | 0,05 |
| NaOH (10 %ige ethan. Lösung) | | − | − | 49,1 | | 46 | 47,3 | 46,5 |
| SZ [mg KOH/g] | | 44 | 47 | 38 | 31 | 38 | 40 | 34 |
| DMEA | (Teile) | 10,5 | 10,5 | − | 9,7 | − | − | − |
| PTGE | " | 192 | − | − | 126 | − | − | − |
| Epikote 812 | " | − | 199 | − | − | − | − | − |
| Epikote 828 | " | − | − | 230 | − | 227 | 246 | 195 |
| Thiodiglykol | " | 1 | 1 | 10,2 | 2,5 | 10 | 10,4 | 19 |
| SZ [mg KOH/g] | | 2,6 | 2,0 | 4 | 3,5 | 3,2 | 2,6 | 5 |
| η 23°C | | 47,5 | 27,5 | 24,9 | 41,3 | 24,4 | 26,2 | 4,3 |

MEHQ = Hydrochinonmonomethylether
DMEA = Dimethylethanolamin
PTGE = Pentaerythrittriglycidylether

4

### Beispiel 8

712 Teile Adipinsäure, 180,5 Teile Phthalsäureanhydrid, 969 Teile Diethylenglykol, 465 Teile Cyclohexan, 6,65 Teile Schwefelsäure und 1,9 Teile hypophosphorige Säure werden zusammengegeben und aufgeheizt. Nachdem 170 Teile Wasser ausgekreist sind, werden 528 Teile Acrylsäure, 271 Teile Cyclohexan, 2,1 Teile Methylhydrochinonmonomethylester, 1,05 Teile Kerobit TBK und 0,07 Teile Phenothiazin zugesetzt. Anschließend wird weiter Wasser ausgekreist (130 Teile in 10 Stunden). Nach destillativer Entfernung des Lösungsmittels weist das Reaktionsgemisch eine Säurezahl von 41 mg KOH/g auf. Es werden nun 12,7 Teile Dimethylethanolamin, 228 Teile PTGE und 9,1 Teile Thiodiglykol zugegeben und die Reaktion bei 105 bis 110 °C fortgeführt. Nach 5 Stunden ist eine SZ von 4,5 mg KOH/g erreicht. Die Viskosität $\eta$ 23 °C beträgt 1,7 Pas.

### Vergleichsbeispiel

Das Polyesterol und das entsprechende Acrylat werden auf die in Beispiel 8 beschriebene Weise hergestellt. Dann wird folgendermaßen weiterverfahren : Zur Neutralisation des Veresterungskatalysators werden 54,3 Teile einer 10 %igen ethanolischen NaOH-Lösung zugegeben, 1 Stunde am Rückfluß erhitzt und dann das Lösungsmittel abgezogen. Anschließend werden aufgrund der Säurezahl von 40 mg KOH/g 257 Teile Epikote 828 und 25 Teile Thiodiglykol zugegeben. Man läßt bei 110 °C reagieren und erhält eine Säurezahl von 3,7 mg KOH/g. Die Viskosität beträgt 1,12 Pas.

Prüfung der Lackeigenschaften

Die gemäß den Beispielen hergestellten Produkte werden nach Verdünnen auf Verarbeitungsviskosität und Zusatz eines Photoinitiators bzw. einer Photoinitiatorkombination in einer 100 μ-Schicht (= Naßfilmstärke) auf Glas aufgetragen und in einem Abstand von ca. 10 cm an einer Quecksilbermitteldrucklampe mit einer Leistung von 80 W/cm vorbeigeführt. Die Bestrahlung erfolgt unter Luft. Der in Tabelle 3 für die Reaktivität angegebene Zahlenwert gibt diejenige Bandgeschwindigkeit an, bei der ein kratzfester Überzug erzielt wird.

### Tabelle 3 : Lackprüfungen

| Polyesteracrylat hergestellt nach Beispiel | (1) | (1) | (2) | (3) | (4) | (5) | (6) | (7) | (8) | Vgl.-beispiel |
|---|---|---|---|---|---|---|---|---|---|---|
| | 100 | 100 | 100 | 100 | 100 | 100 | 100 | 100 | 100 | 100 |
| HDA2 | 65 | – | 65 | 55 | 65 | 59 | 50 | 31 | 17 | 9 |
| TPGDA | – | 90 | – | – | – | – | – | – | – | – |
| BDK | 1,65 | 1,95 | 1,65 | 1,55 | 1,65 | 1,59 | 1,5 | 1,31 | 1,17 | 1,09 |
| BZ | 3,3 | 3,8 | 3,3 | 3,1 | 3,3 | 3,18 | 3,0 | 2,62 | 2,34 | 2,18 |
| MDEA | 4,95 | 5,85 | 4,95 | 4,65 | 4,95 | 4,77 | 4,5 | 3,93 | 3,51 | 3,27 |
| Auslaufzeit (DIN 4, 23°C) | 100 | 98 | 78 | 100 | 85 | 100 | 95 | 98 | 100 | 100 |
| Reaktivität (m/min) | 65 | 45 | 65 | 55 | 65 | 55 | 40 | 35 | 40 | < 5 |
| Bandhärte (DIN 53 157) | 42 | 29 | 39 | 32 | 38 | 31 | 34 | 31 | 42 | – |

HDA 2 = Hexandioldiacrylat
TPGDA = Tripropylenglykoldiacrylat
BDK = Benzildimethylketal
RZ = Benzophenon
MDEA = Methyldiethanolamin

## Patentansprüche

1. Verfahren zur Herstellung von Estern der Acryl- oder Methacrylsäure mit hydroxylgruppenhaltigen organischen Verbindungen, dadurch gekennzeichnet, daß man gesättigte, gegebenenfalls Ethergruppen enthaltende Polyester, die mindestens 2 freie Hydroxylgruppen pro Molekül enthalten, oder Polyether, die

5

mindestens 2 freie Hydroxylgruppen pro Molekül enthalten, wobei diese Polyester oder Polyether durchschnittliche Molekulargewichte $\bar{M}_n$ zwischen 400 und 4 000 aufweisen, mit 100 bis 150 Mol.%, bezogen auf die OH-Gruppen des Polyesters bzw. Polyethers, Acrylsäure oder Methacrylsäure in Gegenwart eines sauren Veresterungskatalysators und mindestens eines Kohlenwasserstoffs, der mit Wasser ein azeotropes Gemisch bildet, sowie geringer Mengen eines Polymerisationsinhibitors unter azeotroper Entfernung des entstehenden Wassers bei erhöhter Temperatur verestert, nach der Veresterung den Kohlenwasserstoff destillativ entfernt und nach Neutralisation des Veresterungskatalysators die restliche Acryl- oder Methacrylsäure mit einer der Säurezahl äquivalenten Menge einer mindestens zwei Epoxidgruppen pro Molekül aufweisenden Epoxidverbindung bis zu einer Säurezahl $\leq$ 5 mg KOH/g umsetzt.

2. Verfahren nach Anspruch 1, dadurch gekennzeichnet, daß man als gesättigte, gegebenenfalls Ethergruppen enthaltende Polyester oder Polyether solche verwendet, die eine OH-Funktionalität zwischen 2 und 3 aufweisen.

3. Verfahren nach Anspruch 1 oder 2, dadurch gekennzeichnet, daß die Hydroxylgruppen der gesättigten, gegebenenfalls Ethergruppen enthaltenden Polyester oder Polyether zu mindestens 85, vorzugsweise 90 bis 95 % mit Acrylsäure oder Methacrylsäure verestert werden.

4. Verfahren nach einem der vorhergehenden Ansprüche, dadurch gekennzeichnet, daß zur Neutralisation des Veresterungskatalysators tertiäre Amine oder wäßrige Lösungen von Alkali- oder Erdalkalihydroxiden verwendet werden.

5. Verfahren nach einem der vorhergehenden Ansprüche, dadurch gekennzeichnet, daß zur Umsetzung der restlichen Acryl- oder Methacrylsäure eine Epoxidverbindung mit drei Epoxidgruppen pro Molekül verwendet wird.

6. Verfahren nach Anspruch 5, dadurch gekennzeichnet, daß als Epoxidverbindung der Triglycidylether des Pentaerythrits verwendet wird.

7. Verfahren nach einem der vorhergehenden Ansprüche, dadurch gekennzeichnet, daß für die Umsetzung der restlichen Acrylsäure oder Methacrylsäure Thiodiglykol als Katalysator verwendet wird.

8. Verfahren nach einem der vorhergehenden Ansprüche, dadurch gekennzeichnet, daß das Umsetzungsprodukt der restlichen Acrylsäure oder Methacrylsäure mit der Epoxidverbindung in einer Menge von 5 bis 25 Gew.% der Gesamtmenge des Veresterungsproduktgemisches erhalten wird.

9. Verwendung der nach einem Verfahren gemäß einem der vorhergehenden Ansprüche hergestellten Produkte in strahlungshärtbaren Überzugsmassen.


**Claims**

1. A process for preparing an ester of acrylic or methacrylic acid with a hydroxyl-containing organic compound, which comprises esterifying a saturated polyester which contains no fewer than 2 free hydroxyl groups per molecule and may contain ether groups or a saturated polyether which contains no fewer than 2 free hydroxyl groups per molecule, this polyester or polyether having an average molecular weight $\bar{M}_n$ of from 400 to 4,000, with from 100 to 150 mol%, based on the OH groups of the polyester or polyether, of acrylic acid or methacrylic acid in the presence of an acidic esterification catalyst and one or more hydrocarbons forming an azeotropic mixture with water and a small amount of a polymerization inhibitor at elevated temperature with azeotropic removal of the resulting water, after the esterification removing the hydrocarbon by distillation and after neutralization of the esterification catalyst reacting the remaining acrylic or methacrylic acid with an amount, equivalent to the acid number, of an epoxy compound having no fewer than two epoxy groups per molecule until an acid number $\leq$ 5 mg of KOH/g is obtained.

2. A process as claimed in claim 1, wherein the saturated polyester, with or without ether groups, or polyether used have an OH functionality of from 2 to 3.

3. A process as claimed in claim 1 or 2, wherein the hydroxyl groups on the saturated polyester, with or without ether groups, or polyether are esterified with acrylic or methacrylic acid to extent of not less than 85 %, preferably from 90 to 95 %.

4. A process as claimed in any of the preceding claims, wherein the esterification catalyst is neutralized with a tertiary amine or an aqueous solution of an alkali metal hydroxide or alkaline earth metal hydroxide.

5. A process as claimed in any of the preceding claims, wherein an epoxy compound having three epoxy groups per molecule is used for converting the remaining acrylic or methacrylic acid.

6. A process as claimed in claim 5, wherein the epoxy compound used is the triglycidyl ether of pentaerythritol.

7. A process as claimed in any of the preceding claims, wherein the conversion of the remaining acrylic acid or methacrylic acid is carried out in the presence of thiodiglycol as a catalyst.

8. A process as claimed in any of the preceding claims, wherein the conversion product of the remaining acrylic acid or methacrylic acid with the epoxy compound is obtained in an amount of from 5 to 25 % by weight of the total amount of the esterification product mixture.

9. A method of using the products prepared in a process as claimed in any of the preceding claims in radiation-curable coating compositions.

**Revendications**

1. Procédé de préparation d'esters de l'acide acrylique ou de l'acide méthacrylique et de composés organiques à groupes hydroxyle, caractérisé en ce que l'on estérifie des polyesters saturés, contenant au moins deux groupes hydroxyle libres par molécule et éventuellement des groupes éther, ou des polyéthers contenant au moins deux groupes hydroxyle libres par molécule, ces polyesters ou polyéthers possédant des poids moléculaires moyens $\bar{M}_n$ compris entre 400 et 4 000, avec 100 à 150 % molaires, par rapport aux groupes hydroxyle du polyester ou du polyéther, d'acide acrylique ou d'acide méthacrylique, en présence d'un catalyseur d'estérification acide, d'au moins un hydrocarbure formant avec l'eau un mélange azéotrope et d'une quantité réduite d'un inhibiteur de la polymérisation, avec élimination par voie azéotropique de l'eau formée à température accrue, l'estérification étant suivie de l'élimination par distillation de l'hydrocarbure et de la neutralisation du catalyseur d'estérification, puis de la réaction de l'acide acrylique ou méthacrylique résiduel avec une proportion équivalente à l'indice d'acide d'un composé époxy avec au moins deux groupes époxy par molécule jusqu'à un indice d'acide inférieur ou égal à 5 mg de KOH par g.

2. Procédé suivant la revendication 1, caractérisé en ce que l'on emploie comme polyéthers ou polyesters saturés, contenant éventuellement des groupes éther, ceux possédant une fonctionnalité hydroxyle comprise entre 2 et 3.

3. Procédé suivant la revendication 1 ou 2, caractérisé en ce que les groupes hydroxyle des polyéthers ou des polyesters saturés, contenant éventuellement des groupes éther, sont estérifiés à raison d'au moins 85 % et de préférence de 90 à 95 % par l'acide acrylique ou l'acide méthacrylique.

4. Procédé suivant l'une des revendications précédentes, caractérisé en ce que la neutralisation du catalyseur d'estérification est réalisée à l'aide d'amines tertiaires ou de solutions aqueuses d'hydroxydes de métaux alcalins ou alcalino-terreux.

5. Procédé suivant l'une des revendications précédentes, caractérisé en ce que l'on fait réagir l'acide acrylique ou méthacrylique résiduel avec un composé époxy contenant trois groupes époxy dans sa molécule.

6. Procédé suivant la revendication 5, caractérisé en ce que le composé époxy est l'éther triglycidylique du pentaérythritol.

7. Procédé suivant l'une des revendications précédentes, caractérisé en ce que le catalyseur pour la réaction de l'acide acrylique ou de l'acide méthacrylique résiduel est le thiodiglycol.

8. Procédé suivant l'une des revendications précédentes, caractérisé en ce que le produit de la réaction de l'acide acrylique ou méthacrylique résiduel et du composé époxy constitue entre 5 et 25 % en poids du poids total du mélange des produits réactionnels de l'estérification.

9. Utilisation des produits obtenus par un procédé suivant l'une des revendications précédentes dans des matières d'enduction durcissables par un rayonnement.